# EUROPEAN PATENT APPLICATION

(11) **EP 4 691 425 A1**
(43) Date of publication of application: **11.02.2026**
(21) Application number: 24816899.9
(22) Date of filing: 11.10.2024
(51) Int. Cl.: A61F 2/30, C23C 4/08

(54) **JOINT PROSTHESIS IMPLANT AND PROCESSING METHOD THEREFOR**

(30) Priority: 30.05.2024 CN 202410692379
(71) Applicant: Beijing AK Medical Co., Ltd., 102200, Beijing (CN)
(72) Inventor: LI, Xinyu, Beijing 102200 (CN); ZHANG, Weiping, Beijing 102200 (CN)
(74) Representative: Studio Torta S.p.A.
(86) International application number: PCT/CN2024/124257
(87) International publication number: WO 2025/246127

(57) **Abstract**

The invention provides a joint prosthesis implant and a processing method for the joint prosthesis implant. The joint prosthesis implant includes: a ceramic substrate, wherein the ceramic substrate includes a friction surface and a connection surface; and a metal coating connected to the connection surface, wherein an interface is formed between the metal coating and the connection surface. The connection surface of the ceramic substrate is provided with a first locking hook part extending into the metal coating, and the metal coating is provided with a second locking hook part extending into the ceramic substrate. The first locking hook part and the second locking hook part are cooperated with each other to form an interlocking structure. The technical solution of the invention effectively solves a problem that a metal coating of a joint prosthesis implant is likely to fall off in the related art.

## Description

### Cross-Reference to Related Application

The invention claims the priority to the Chinese Patent Application No. 202410692379.0, filed on May 30, 2024 and entitled "Joint prosthesis implant and processing method for Joint Prosthesis Implant", which is incorporated herein by reference in its entirety.

### Technical Field

The invention relates to the field of prostheses, and particularly relates to a joint prosthesis implant and a processing method for the joint prosthesis implant.

### Background

At present, a total hip prosthesis system is composed of an acetabular cup, a ball end, a lining and a femoral stem. A physiological structure of a hip joint is reconstructed with a complex mechanical combined structure. This prosthesis system is highly satisfactory for current primary hip replacement. With the gradual progress in medical technology, increasing attention has been paid to diagnosis, treatment and discovery of early hip joint lesions. Nowadays, hip joint lesions (such as arthritis or femoral head necrosis) of patients can be discovered through disease screening at an early stage. Especially, younger patients typically have stronger needs for physical activity, so routine simple reconstruction of hip joint functions can no longer meet their needs. Further, postoperative quality of life of the patients having stronger needs for physical activity is severely restricted by subsequent clinical problems such as loosening and dislocation of prostheses.

In the related art, a ceramic material, as an excellent bio-inert material, has biosecurity clinically verified for nearly 20 years. The ceramic material is used as an optimal wear-resistant material for an articular surface due to its excellent wear resistance, and can provide patients with better physiological movement functions of hip joints. Regarding the above structure, a metal transition member or a bone cement filling layer is generally required between a ceramic prosthesis (such as a ceramic acetabular cup and a ceramic ball end) and a physiological bone interface, so as to fix the ceramic prosthesis to a bone bed.

An integral ceramic acetabular cup is an ideal solution, by which an ideal joint friction surface and a bioactive interface that can achieve perfect osseointegration with a physiological bone coexist on a prosthesis. This poses great challenges to manufacturing processes. An alternative solution is to apply a titanium or tantalum metal layer to a contact part of a ceramic joint prosthesis and a physiological bone through a high-temperature plasma spraying technology. Based on biological activity characteristics of titanium, tantalum or other metals having good biocompatibility, osseointegration between the prosthesis and the physiological bone can be formed after implantation in a human body. A joint friction surface of a ceramic joint prosthesis can become an ideal smooth friction surface through high-temperature sintering and precision processing. However, in a case that titanium or tantalum metal powder or other metals having good biocompatibility are sprayed through high-temperature plasma onto a dense ceramic surface on an osseointegration interface by a general surface spraying technology, bonding strength of a coating will be ultralow because of characteristic differences between metal and ceramic, and the coating is likely to fall off.

### Summary

A main objective of the invention is to provide a joint prosthesis implant and a processing method for the joint prosthesis implant, so as to solve a problem that a metal coating of a joint prosthesis implant is likely to fall off in the related art.

In order to achieve the above objective, one aspect of the invention provides a joint prosthesis implant. The joint prosthesis implant includes: a ceramic substrate, wherein the ceramic substrate includes a friction surface and a connection surface; and a metal coating connected to the connection surface, wherein an interface is formed between the metal coating and the connection surface. The connection surface of the ceramic substrate is provided with a first locking hook part extending into the metal coating. The metal coating is provided with a second locking hook part extending into the ceramic substrate. The first locking hook part and the second locking hook part are cooperated with each other to form an interlocking structure.

Further, the first locking hook part includes a first hook body part and a second hook body part that are connected with each other. The first hook body part is located between the ceramic substrate and the second hook body part. A first included angle is formed between the first hook body part and the interface. A second included angle is formed between the first hook body part and the interface. The second included angle is smaller than the first included angle.

Further, an end of the second locking hook part is located inside a root of the first locking hook part.

Further, a surface of the metal coating away from the ceramic substrate is provided with a recess at a position corresponding to an end of the first locking hook part.

Further, a thickness of the ceramic substrate gradually decreases in a direction from a center of the ceramic substrate to a periphery of the ceramic substrate.

Further, the metal coating includes a central zone and a plurality of annular zones located outside the central zone. A thickness variation of the ceramic substrate corresponding to a same annular zone is smaller than or equal to 0.2 mm.

Another aspect of the invention provides a processing method for a joint prosthesis implant. The processing method is configured to manufacture the joint prosthesis implant. The processing method includes: conducting spraying on a connection surface of a ceramic substrate, so as to form a metal coating on the connection surface; and forming a first locking hook part extending into the metal coating at the connection surface of the ceramic substrate, and forming a second locking hook part extending into the ceramic substrate at the metal coating. The first locking hook part and the second locking hook part are cooperated with each other to form an interlocking structure.

Further, the forming a first locking hook part extending into the metal coating at the connection surface of the ceramic substrate, and forming a second locking hook part extending into the ceramic substrate at the metal coating include: processing the metal coating and the ceramic substrate by a first laser, such that the connection surface is provided with a first protrusion and a second protrusion that extend into the metal coating and are provided in a spaced manner, and the metal coating is provided with a third protrusion extending into the ceramic substrate; and processing the first protrusion by a second laser, such that the first protrusion is obliquely provided towards the second protrusion, and forming a fourth protrusion on an outer surface of the metal coating. A preset included angle is formed between an incident direction of the second laser and an incident direction of the first laser. The first protrusion obliquely provided constitutes the first locking hook part. The third protrusion constitutes the second locking hook part.

Further, after the processing the first protrusion by a second laser, the processing method further includes: processing the first protrusion obliquely provided by a third laser, such that an end of the first protrusion moves towards the ceramic substrate.

Further, the incident direction of the first laser is a normal direction of the ceramic substrate; and/or the preset included angle between the incident direction of the second laser and the incident direction of the first laser is greater than or equal to 20° and smaller than or equal to 45°.

Further, energy of the first laser is greater than that of the second laser, and an area of the first laser is greater than that of the second laser; and/or an area of the third laser is greater than an area of the second laser.

Further, the conducting spraying on a connection surface of a ceramic substrate, so as to form a metal coating on the connection surface includes: conducting spraying on a center of the connection surface so as to obtain a central zone; and conducting spraying outwards from the central zone so as to sequentially obtain a plurality of annular zones located outside the central zone. A thickness variation of the ceramic substrate corresponding to a same annular zone is smaller than or equal to 0.2 mm. The central zone and the plurality of annular zones constitute the metal coating.

Further, the conducting spraying outwards from the central zone so as to sequentially obtain a plurality of annular zones located outside the central zone includes: shortening spraying time and/or interval time of the plurality of annular zones sequentially.

According to the technical solution of the invention, the joint prosthesis implant includes: the ceramic substrate and the metal coating. The ceramic substrate includes the friction surface and the connection surface. The metal coating is connected to the connection surface. The interface is formed between the metal coating and the connection surface. The connection surface of the ceramic substrate is provided with the first locking hook part extending into the metal coating. The metal coating is provided with the second locking hook part extending into the ceramic substrate. The first locking hook part and the second locking hook part are cooperated with each other to form the interlocking structure. By cooperating the first locking hook part and the second locking hook part to form the interlocking structure, bonding strength between the metal coating and the ceramic substrate can be effectively improved, such that the metal coating is unlikely to be separated from the ceramic substrate. Thus, the technical solution of the invention effectively solves a problem that a metal coating of a joint prosthesis implant is likely to fall off in the related art.

### Brief Description of the Drawings

The accompanying drawings, constituting part of the description, are used to provide further understanding of the invention, and illustrative embodiments and their description of the invention serve to explain the invention instead of improperly limiting the invention. In the drawings:
Fig. 1 shows a schematic diagram of a cutaway view of an embodiment of a joint prosthesis implant according to the invention;
Fig. 2 shows a schematic enlarged diagram of A of the joint prosthesis implant in Fig. 1;
Fig. 3 shows a schematic perspective view of a ceramic substrate of the joint prosthesis implant in Fig. 1;
Fig. 4 shows a schematic diagram of a cutaway view of the ceramic substrate in the Fig. 3;
Fig. 5 shows a schematic structural diagram of a metal coating of the joint prosthesis implant in Fig. 1;
Fig. 6 shows a schematic flow diagram of an embodiment of a processing method for a joint prosthesis implant according to the invention;
Fig. 7 shows a schematic diagram of spraying time and interval time of a plurality of annular zones in the processing method of Fig. 6;
Fig. 8 shows a schematic diagram of a laser path for processing a metal coating and a ceramic substrate by means of a first laser in the processing method of Fig. 6;
Fig. 9 shows a schematic diagram of another laser path for processing a metal coating and a ceramic substrate by means of a first laser in the processing method of Fig. 6;
Fig. 10 shows a schematic diagram of processing a metal coating and a ceramic substrate by means of a first laser in the processing method of Fig. 6;
Fig. 11 shows a schematic structural diagram of a cutaway view of a metal coating and a ceramic substrate that are processed by a first laser;
Fig. 12 shows a schematic diagram of processing a first protrusion by means of a second laser in the processing method of Fig. 6;
Fig. 13 shows a schematic structural diagram of a cutaway view of a metal coating and a ceramic substrate that are processed by a second laser;
Fig. 14 shows a schematic diagram of an oblique first protrusion processed by third laser in another embodiment of a processing method for a joint prosthesis implant according to the invention;
Fig. 15 shows a schematic structural diagram of a cutaway view of a metal coating and a ceramic substrate that are processed by a third laser; and
Fig. 16 shows a schematic diagram of a first included angle between a first hook body part and an interface and a second included angle between a second hook body part and the interface of the joint prosthesis implant in Fig. 1.

The above figures include the following reference numerals:
10: ceramic substrate; 11: friction surface; 111: recess; 12: connection surface; 13: first locking hook part; 131: first hook body part; 132: second hook body part; 133: root; 20: metal coating; 21: second locking hook part; 211: end; 22: central zone; 23: annular zone; 30: interface.

### Detailed Description of the Embodiments

Technical solutions of embodiments of the invention will be clearly and completely described below in conjunction with the accompanying drawings of the embodiments of the invention. Obviously, the embodiments described are merely some embodiments rather than all the embodiments of the invention. The following description of at least one illustrative embodiment is merely illustrative in nature and in no way serves as any limitation of the invention and its application or use. On the basis of the embodiments of the invention, all other embodiments obtained by those of ordinary skill in the art without making creative efforts fall within the protection scope of the invention.

It should be noted that the terms used herein are merely for describing the detailed description of embodiments and are not intended to limit illustrative embodiments according to the invention. As used herein, the singular is also intended to include the plural unless otherwise specified in the context. In addition, it should be understood that the terms "involve" and/or "comprise" or "include" used in the description indicate the presence of features, steps, operations, devices, assemblies, and/or combinations thereof.

It should be noted that the relative arrangement, numerical expressions and numerical values of components and steps described in the embodiments do not limit the scope of the invention unless otherwise specified. Moreover, it should be understood that for convenience of description, dimensions of each part shown in the drawings are not drawn according to a real proportional relation. Technologies, methods and apparatuses known to those of ordinary skill in the related art may not be discussed in detail, but in appropriate cases, they should be regarded as part of the description. In all instances shown and discussed herein, any specific value should be interpreted as illustrative only, instead of being limitative. Thus, different values are possible in other instances of the illustrative embodiments. It should be noted that like numerals and letters denote like items in the following accompanying drawings, and thus, once an item is defined in one accompanying drawing, it does not need to be further discussed in the subsequent accompanying drawings.

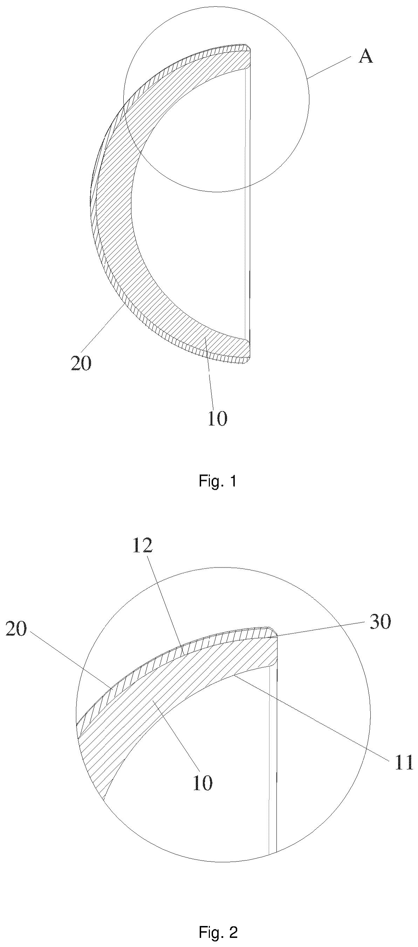

As shown in Figs. 1, 2, and 13, a joint prosthesis implant of the embodiment includes: a ceramic substrate 10 and a metal coating 20. The ceramic substrate 10 includes a friction surface 11 and a connection surface 12. The metal coating 20 is connected to the connection surface 12. An interface 30 is formed between the metal coating 20 and the connection surface 12. The connection surface 12 of the ceramic substrate 10 is provided with a first locking hook part 13 extending into the metal coating 20. The metal coating 20 is provided with a second locking hook part 21 extending into the ceramic substrate 10. The first locking hook part 13 and the second locking hook part 21 are cooperated with each other to form an interlocking structure.

Through the technical solution of the embodiment, by cooperating the first locking hook part 13 and the second locking hook part 21 to form the interlocking structure, bonding strength between the metal coating 20 and the ceramic substrate 10 can be effectively improved, such that the metal coating 20 is unlikely to be separated from the ceramic substrate 10. Thus, the technical solution of the invention effectively solves a problem that a metal coating of a joint prosthesis implant is likely to fall off in the related art.

As a feasible embodiment, the metal coating 20 may be a titanium coating or a tantalum coating.

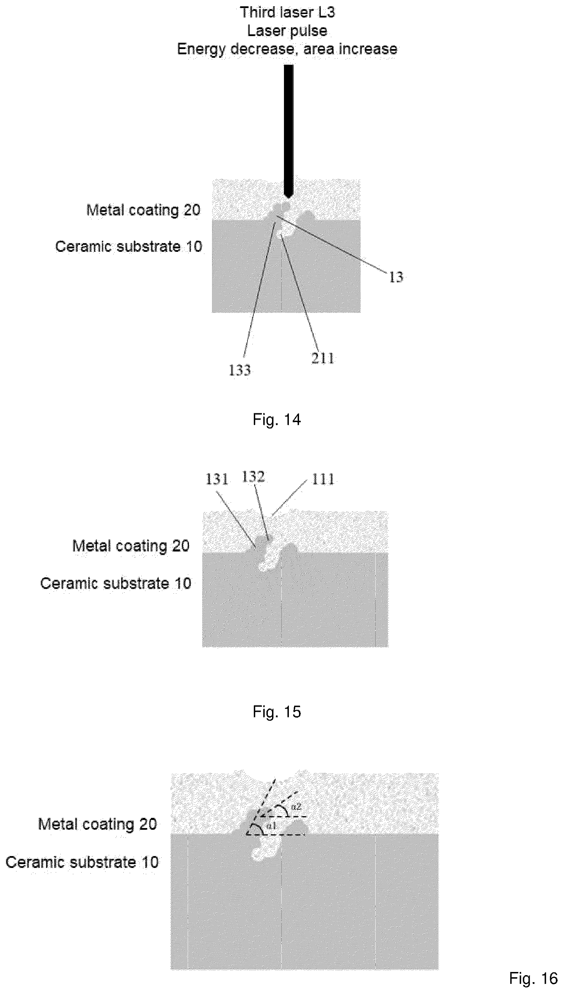

As shown in Figs. 15 and 16, the first locking hook part 13 includes a first hook body part 131 and a second hook body part 132 that are connected with each other. The first hook body part 131 is located between the ceramic substrate 10 and the second hook body part 132. A first included angle α1 is formed between the first hook body part 131 and the interface 30. A second included angle α2 is formed between the second hook body part 132 and the interface 30. The second included angle α2 is smaller than the first included angle α1. The above structure is conducive to further improvement in bonding strength between the metal coating 20 and the ceramic substrate 10. As shown in Fig. 16, the first hook body part 131 is of an irregular protruding structure. The first included angle α1 is essentially an included angle between a center line of the first hook body part 131 and the interface 30. Similarly, the second hook body part 132 is of an irregular protruding structure. The second included angle α2 is essentially an included angle between a center line of the second hook body part 132 and the interface 30. Specific molding methods for the first hook body part 131 and the second hook body part 132 will be described in detail in a section of a processing method.

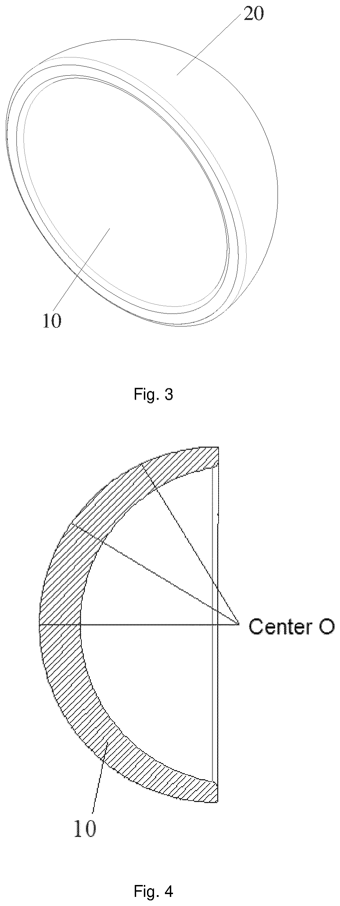

As shown in Fig. 14, an end 211 of the second locking hook part 21 is located inside a root 133 of the first locking hook part 13. The above structure enables the end 211 of the second locking hook part 21 to be limited by the root 133 of the first locking hook part 13, so as to be conducive to further improvement in bonding strength between the metal coating 20 and the ceramic substrate 10. It should be noted that the "inside" means that the end 211 of the second locking hook part 21 is closer to a center of the ceramic substrate 10 than the root 133 of the first locking hook part 13 (the center O is shown in Fig. 4).

As shown in Fig. 15, a surface of the metal coating 20 away from the ceramic substrate 10 is provided with a recess 111 at a position corresponding to an end of the first locking hook part 13. A structure of the recess 111 enables the surface of the metal coating 20 away from the ceramic substrate 10 to be smoother and can improve smoothness of the surface of the metal coating 20 away from the ceramic substrate 10.

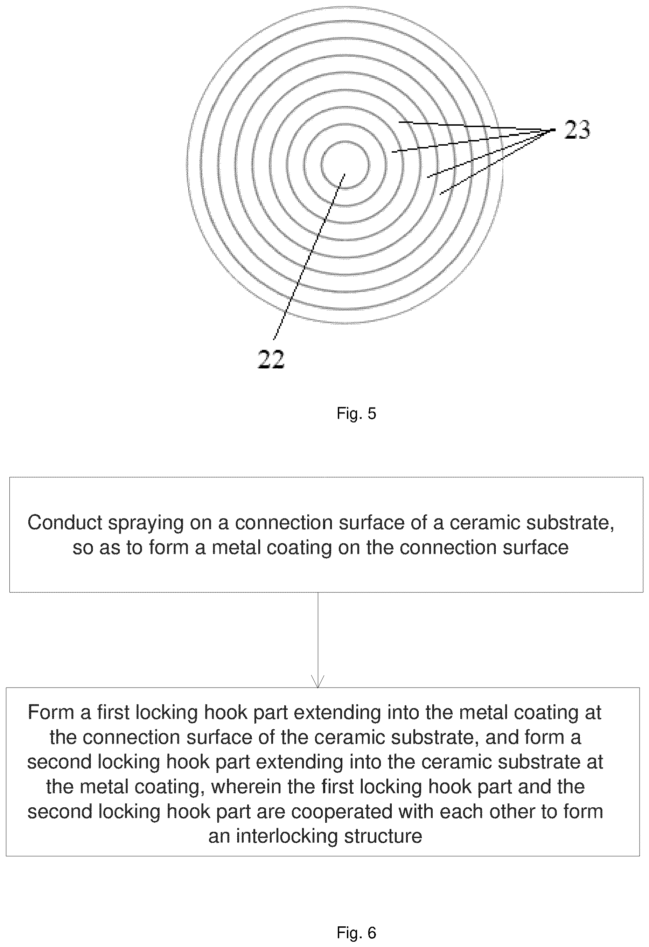

As shown in Figs. 4 and 5, a thickness of the ceramic substrate 10 gradually decreases in a direction from a center of the ceramic substrate 10 to a periphery of the ceramic substrate 10. The above structure can match a partitioned processing method for the metal coating 20, and the ceramic substrate 10 is designed in a form of variable thickness according to a thermal stress state, such that gradual release of thermal stress is facilitated in a spraying process. In this way, the interface 30 between the processed metal coating 20 and the ceramic substrate 10 has a common thermal deformation rate, which is conducive to further improvement in bonding strength between the metal coating 20 and the ceramic substrate 10.

As shown in Figs. 1, 2, and 5, the metal coating 20 includes a central zone 22 and a plurality of annular zones 23 located outside the central zone 22. A thickness variation of the ceramic substrate 10 corresponding to a same annular zone 23 is smaller than or equal to 0.2 mm. During processing of the metal coating 20, spraying is conducted on a center of the connection surface 12 so as to obtain the central zone 22, and then spraying is conducted outwards from the central zone 22 so as to sequentially obtain the plurality of annular zones 23 located outside the central zone 22. The central zone 22 and the plurality of annular zones 23 constitute the metal coating 20. The interface 30 between the metal coating 20 obtained through the above method and the ceramic substrate 10 has a common thermal deformation rate, which is conducive to further improvement in bonding strength between the metal coating 20 and the ceramic substrate 10.

In the embodiment, the joint prosthesis implant is an acetabular cup. In other embodiments, the joint prosthesis implant may also be another part of a prosthesis implant.

The invention further provides a processing method for a joint prosthesis implant. The processing method is configured to manufacture the joint prosthesis implant. As shown in Figs. 6-15, an embodiment of the processing method of the invention includes the following steps:
spraying is conducted on a connection surface 12 of a ceramic substrate 10, so as to form a metal coating 20 on the connection surface 12; and
a first locking hook part 13 extending into the metal coating 20 is formed at the connection surface 12 of the ceramic substrate 10, and a second locking hook part 21 extending into the ceramic substrate 10 is formed at the metal coating 20, wherein the first locking hook part 13 and the second locking hook part 21 are cooperated with each other to form an interlocking structure. The interlocking structure may also be referred to as a mutually locked structure.

According to the technical solution of the embodiment, by cooperating the first locking hook part 13 and the second locking hook part 21 to form the interlocking structure, bonding strength between the metal coating 20 and the ceramic substrate 10 can be effectively improved, such that the metal coating 20 is unlikely to be separated from the ceramic substrate 10. Thus, the technical solution of the invention effectively solves a problem that a metal coating of a joint prosthesis implant is likely to fall off in the related art.

In the embodiment, a sprayed zone, that is, the metal coating 20, is selectively scanned through a laser pulse cladding technology, and a penetration depth and laser energy are controlled, such that a mutually locked structure is formed between the metal coating 20 and the ceramic substrate 10, and bonding strength of the metal coating 20 is enhanced.

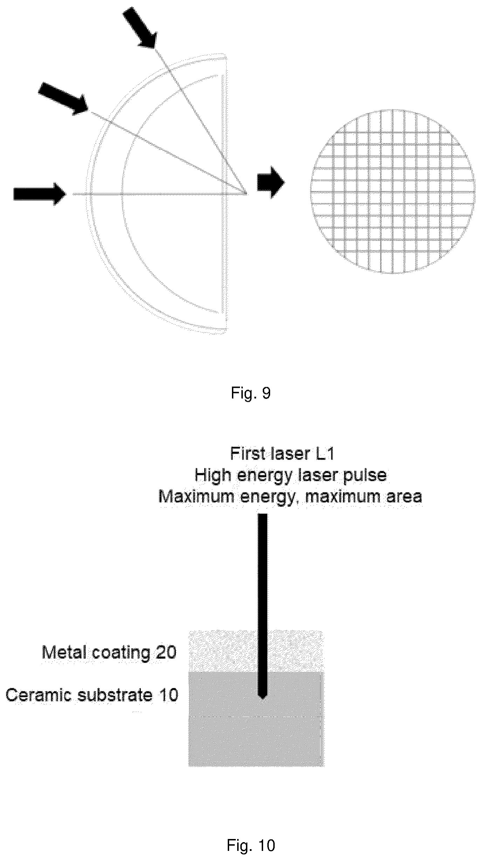

As shown in Figs. 10-13, the steps that the first locking hook part 13 extending into the metal coating 20 is formed at the connection surface 12 of the ceramic substrate 10, and the second locking hook part 21 extending into the ceramic substrate 10 is formed at the metal coating 20 include the following steps:
the metal coating 20 and the ceramic substrate 10 are processed by a first laser L1, such that the connection surface 12 is provided with a first protrusion M1 and a second protrusion M2 that extend into the metal coating 20 and are provided in a spaced manner, and the metal coating 20 is provided with a third protrusion M3 extending into the ceramic substrate 10; and
the first protrusion M1 is processed by a second laser L2, such that the first protrusion M1 is obliquely provided towards the second protrusion M2, and a fourth protrusion M4 is formed on an outer surface of the metal coating 20. A preset included angle α3 is formed between an incident direction of the second laser L2 and an incident direction of the first laser L1. The first protrusion M1 obliquely provided constitutes the first locking hook part 13. The third protrusion M3 constitutes the second locking hook part 21.

In the method, the first protrusion M1 processed by the second laser L2 is obliquely arranged towards the second protrusion M2, which is conducive to further improvement in bonding strength between the metal coating 20 and the ceramic substrate 10.

In order to further improve a locking effect of the interlocking structure constituted by the first locking hook part 13 and the second locking hook part 21, after the first protrusion is processed by the second laser L2, the processing method further includes the following step: the first protrusion M1 obliquely provided is processed by a third laser L3, such that an end of the first protrusion M1 moves towards the ceramic substrate 10.

In the method, the first protrusion M1 processed by the third laser L3 constitutes the first hook body part 131 and the second hook body part 132. Thus, the second hook body part 132 is obliquely provided towards the ceramic substrate 10 relative to the first hook body part 131. That is, processing of the third laser L3 can further narrow a locking opening between materials near the interface 30. In this way, further improvement in bonding strength between the metal coating 20 and the ceramic substrate 10 is facilitated. In the above step, the fourth protrusion M4 is processed into the recess 111 by the third laser L3.

As a preferable embodiment, the incident direction of the first laser L1 is a normal direction of the ceramic substrate 10. The preset included angle α3 between the incident direction of the second laser L2 and the incident direction of the first laser L1 is greater than or equal to 20° and smaller than or equal to 45°. The preset included angle α3 between the incident direction of the second laser L2 and the incident direction of the first laser L1 may be 25°, 30°, 35°, 40° or 45°. Clearly, the preset included angle may not be limited to the above angle range.

Preferably, an incident direction of the third laser L3 is parallel to the incident direction of the first laser L1, or an included angle between the incident direction of the third laser and the incident direction of the first laser is smaller than the preset included angle α3 between the incident direction of the second laser L2 and the incident direction of the first laser L1.

In the processing method of the embodiment, energy of the first laser L1 is greater than that of the second laser L2, and an area of the first laser L1 is greater than that of the second laser L2. An area of the third laser L3 is greater than that of the second laser L2. The greater laser energy, the greater the penetration depth, and the more remarkable an interface interlocking effect. The greater a laser area, the greater a molten pool area, and the more remarkable a surface smoothing effect.

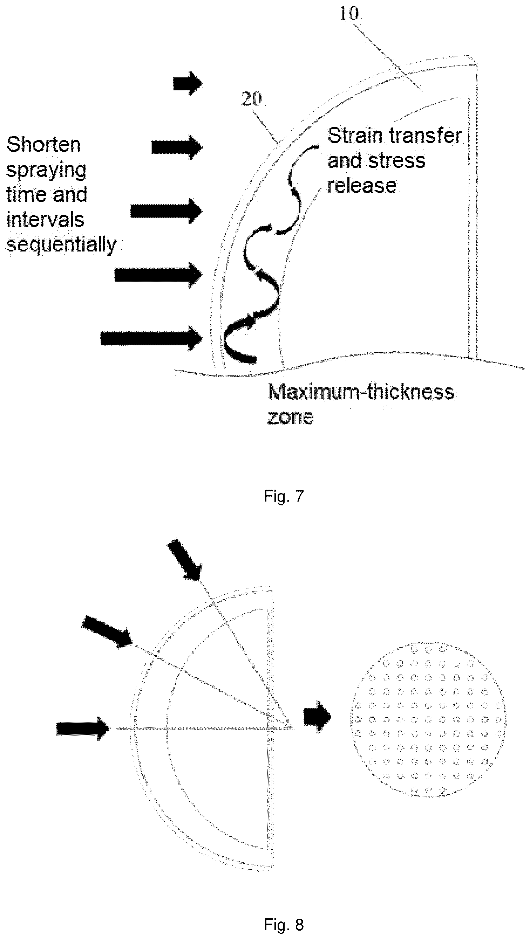

As shown in Fig. 7, the step that spraying is conducted on the connection surface 12 of the ceramic substrate 10, so as to form the metal coating 20 on the connection surface 12 includes the following steps: spraying is conducted on the center of the connection surface 12 so as to obtain the central zone 22; and spraying is conducted outwards from the central zone 22 so as to sequentially obtain the plurality of annular zones 23 located outside the central zone 22. The thickness variation of the ceramic substrate 10 corresponding to the same annular zone 23 is smaller than or equal to 0.2 mm. The central zone 22 and the plurality of annular zones 23 constitute the metal coating 20.

In the above step, the metal coating 20 of the ceramic substrate 10 is attached to the ceramic substrate 10 through a partitioned high-temperature spraying process.

As shown in Fig. 7, the step that spraying is conducted outwards from the central zone 22 so as to sequentially obtain the plurality of annular zones 23 located outside the central zone 22 includes the following step: spraying time and/or interval time of the plurality of annular zones 23 are/is shortened sequentially. High-temperature spraying time is controlled to be shortened from the central zone 22 to the outside. Spraying is conducted on the central zone for 2 s-2.5 s, and spraying time is gradually reduced outwards with a gradient of 0.2 s-0.3 s. The interval time is controlled. After spraying is conducted on the central zone 22, spraying is conducted on a next zone after an interval of 2 s-4 s. In a spraying process of outer zones, the interval time is reduced sequentially with a gradient of 0.3 s-0.5 s. Thus, internal temperature stress is controlled to be close to a uniform state.

The interface 30 between the metal coating 20 obtained through the above method and the ceramic substrate 10 has a common thermal deformation rate, which is conducive to further improvement in bonding strength between the metal coating 20 and the ceramic substrate 10.

An innovation of the invention lies in that by applying a processing method, a surface metal-sprayed coating based on the ceramic substrate may obtain extremely high coating bonding strength, such that the coating can have more stable and reliable performance. On the basis of the processing method, an all-ceramic metal-coated acetabular cup and a ceramic ball end can be implemented. Clearly, other implants requiring osseointegration functions based on the ceramic substrate can also be implemented.

The preferred embodiments of the invention will be described below in conjunction with Figs. 1-16.

On the basis of a design of an acetabular cup having a ceramic substrate variable in thickness, the embodiment uses processes of partitioned spraying and laser pulse cladding and interlocking to conduct cladding and enhance strength of the coating in a local, partitioned or integral manner, such that a laser selective melting ceramic-coating process is implemented. A specific implementation form is as follows:
The acetabular cup is integrally designed with a smooth inner surface and a variable-thickness interface. A change form of the thickness is related to a sequence of spraying zones. In existing spraying processes, spraying is conducted on an entire to-be-sprayed surface, and generally on a rotating or fixed platform. The process generally does not need to consider a spraying sequence and a positional relation. In a case of a metal substrate, for a metal coating sprayed onto the metal substrate, the substrate and the coating have similar thermal expansion rates. Thus, interlayer peeling caused by different shrinkage rates cannot occur after cooling. Thermal conductivity of a ceramic material is slightly lower than that of metal, and a thermal deformation rate of the ceramic material is smaller than that of metal. Thermal expansion coefficients of titanium, tantalum and ceramics are 8.6×10⁻⁶/°C, 6.6×10⁻⁶/°C and 9.6×10⁻⁶/°C, respectively. Thermal deformation performance of a base material is slightly higher than that of a technically feasible metal coating material. Considering that high-temperature plasma spraying may increase a local temperature in a coating cladding process, thermal deformation of the material has to be fully considered to avoid great internal stress on the base material due to rapid shrinkage of the coating material.

Thus, in the embodiment, a structural form with the variable thickness is designed, and thermal deformation rates of the coating material and the base material are adjusted to be consistent through changes of heat transfer and expansion rates of the material caused by thickness change, in cooperation with a form of selective spraying. Further, in order to ensure firm combination of a coating part and the base material, the invention uses a form of laser pulse, and an interface between a base and a coating is penetrated when the coating is in a semi-molten state, such that a layer interlocking structure is formed, and bonding strength of the coating is greatly improved. In the invention, with an acetabular cup as an instance, illustration is provided as follows:
the acetabular cup and its interface variable in thickness are shown in Fig. 1. In a hemispherical acetabular cup structure, a feasible thickness change is to reduce a thickness along an extreme top to an edge sequentially. The thickness change corresponds to a spraying zone and a spraying sequence.

According to changes of the thickness of a ceramic acetabular cup, zones are partitioned along rings from a center of an outer surface of the extreme top to the edge. Although a wall thickness of the acetabular cup changes continuously, the thickness variation of a same zone is smaller than 0.2 mm. The thickness is computed according to a normal direction from a center O to a curved surface. As shown in Fig. 7.

After partitioning, in a spraying process, spraying is conducted sequentially from an inner ring to an outer ring. The entire acetabular cup begins to deform from a high-temperature zone at a bottom of the acetabular cup, and heat is transferred from the center to a periphery. A nozzle is controlled to conduct spraying in the outer ring sequentially, and spraying intervals between the zones are shortened sequentially. Finally, an interface material between the acetabular cup and a coating has a common thermal deformation rate, as shown in Fig. 7.

After a spraying operation is finished, the coating is scanned by high-energy pulsed laser beams. A scanning path may be designed as a dot matrix, a grid, a spiral line and other regular or irregular point-line-surface combinations as required. Laser output power is controlled at an energy state slightly larger than a depth of the metal coating, such that the scanned metal coating and a surface of a ceramic base below the coating constitute a tiny molten pool, and coated metal and a ceramic surface structure of a base are re-stirred and integrated to form a mutually locked structure. The structure can further integrate the entire coating with the base material, so as to improve bonding strength of the entire coating. If necessary, the coating surface may be fully scanned and integrated. Figs. 8 and 9 show two laser paths.

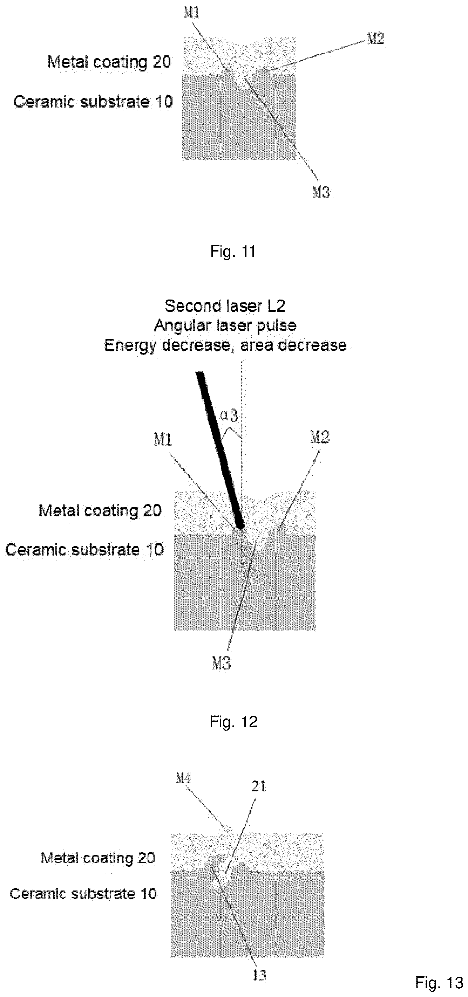

In a process of controlling the laser path and an incident direction, the mutually locked structure for penetrating the interface between the metal coating and the ceramic substrate is obtained through multi-laser beam deflection. Firstly, as shown in Figs. 10 and 11, a molten pool foundation is generated by high-energy laser, that is, a first laser L1. An energy transmission intensity is controlled to be slightly higher than a thickness of the metal coating. Temperatures of the coating and the base material are raised in a certain range, such that a semi-molten state is formed. Then, as shown in Figs. 12 and 13, an incident angle of laser is slightly adjusted to deflect from an initial angle, and a second laser L2 is obtained. The second laser L2 disrupts an interlayer sequence in the molten pool so as to play a stirring role. Angular penetration causes deformation of the molten pool, and further a locking opening between materials near the interface is narrowed. Finally, as shown in Figs. 14 and 15, a laser angle is readjusted, a third laser L3 is obtained, and an irradiation area is increased. The third laser L3 compacts a first locking hook part 13 with light pressure energy, and a material locking structure is further formed between the interfaces. Meanwhile, an asymmetric structure on the surface caused by deformation of the molten pool in the previous step is smoothed.

It should be noted that when a burr structure needs to be evenly distributed on an outer surface, radial laser and variable incident-angle laser may also be used alternately, so as to reduce the step of smooth decoration with light pressure, such that an extremely high burr protruding structure is obtained, and the outer surface having extremely high roughness is obtained.

Through the method, by controlling thermal deformation in a process of ceramic spraying and strengthening with laser pulse after spraying, a material having the ceramic substrate and the metal coating and having extremely high strength may be obtained. In the invention, laser is applied in a form of dot matrix or square matrix scanning. Other similar methods fall within the technical scope of the invention, such that bonding performance of a coating structure can be improved in a certain area.

What are described above are merely preferred embodiments of the invention and are not intended to limit the invention, and various changes and modifications can be made by those skilled in the art. Any modifications, equivalent substitutions, improvements, etc. made within the spirit and principle of the invention should fall within the protection scope of the invention.

## Claims

1. A joint prosthesis implant, comprising:
a ceramic substrate (10), wherein the ceramic substrate (10) comprises a friction surface (11) and a connection surface (12); and
a metal coating (20) connected to the connection surface (12), wherein an interface (30) is formed between the metal coating (20) and the connection surface (12), wherein
the connection surface (12) of the ceramic substrate (10) is provided with a first locking hook part (13) extending into the metal coating (20), the metal coating (20) is provided with a second locking hook part (21) extending into the ceramic substrate (10), and the first locking hook part (13) and the second locking hook part (21) are cooperated with each other to form an interlocking structure.

2. The joint prosthesis implant according to claim 1, wherein the first locking hook part (13) comprises a first hook body part (131) and a second hook body part (132) that are connected with each other, the first hook body part (131) is located between the ceramic substrate (10) and the second hook body part (132), a first included angle (α1) is formed between the first hook body part (131) and the interface (30), a second included angle (α2) is formed between the second hook body part (132) and the interface (30), and the second included angle (α2) is smaller than the first included angle (α1).

3. The joint prosthesis implant according to claim 1, wherein an end (211) of the second locking hook part (21) is located inside a root (133) of the first locking hook part (13).

4. The joint prosthesis implant according to claim 1, wherein a surface of the metal coating (20) away from the ceramic substrate (10) is provided with a recess (111) at a position corresponding to an end of the first locking hook part (13).

5. The joint prosthesis implant according to claim 1, wherein a thickness of the ceramic substrate (10) gradually decreases in a direction from a center of the ceramic substrate (10) to a periphery of the ceramic substrate (10).

6. The joint prosthesis implant according to claim 1, wherein the metal coating (20) comprises a central zone (22) and a plurality of annular zones (23) located outside the central zone (22), and a thickness variation of the ceramic substrate (10) corresponding to a same annular zone (23) is smaller than or equal to 0.2 mm.

7. A processing method for a joint prosthesis implant, configured to manufacture the joint prosthesis implant according to any one of claims 1-6, comprising:
conducting spraying on a connection surface (12) of a ceramic substrate (10), so as to form a metal coating (20) on the connection surface (12); and
forming a first locking hook part (13) extending into the metal coating (20) at the connection surface (12) of the ceramic substrate (10), and forming a second locking hook part (21) extending into the ceramic substrate (10) at the metal coating (20), wherein the first locking hook part (13) and the second locking hook part (21) are cooperated with each other to form an interlocking structure.

8. The processing method according to claim 7, wherein the forming a first locking hook part (13) extending into the metal coating (20) at the connection surface (12) of the ceramic substrate (10), and forming a second locking hook part (21) extending into the ceramic substrate (10) at the metal coating (20) comprise:
processing the metal coating (20) and the ceramic substrate (10) by a first laser (L1), such that the connection surface (12) is provided with a first protrusion (M1) and a second protrusion (M2) that extend into the metal coating (20) and are provided in a spaced manner, and the metal coating (20) is provided with a third protrusion (M3) extending into the ceramic substrate (10); and
processing the first protrusion (M1) by a second laser (L2), such that the first protrusion (M1) is obliquely provided towards the second protrusion (M2), and forming a fourth protrusion (M4) on an outer surface of the metal coating (20), wherein a preset included angle (α3) is formed between an incident direction of the second laser (L2) and an incident direction of the first laser (L1), wherein
the first protrusion (M1) obliquely provided constitutes the first locking hook part (13), and the third protrusion (M3) constitutes the second locking hook part (21).

9. The processing method according to claim 8, wherein after the processing the first protrusion (M1) by a second laser (L2), the processing method further comprises:
processing the first protrusion (M1) obliquely provided by a third laser (L3), such that an end of the first protrusion (M1) moves towards the ceramic substrate (10).

10. The processing method according to claim 8, wherein
the incident direction of the first laser (L1) is a normal direction of the ceramic substrate (10); and/or
the preset included angle (α3) between the incident direction of the second laser (L2) and the incident direction of the first laser (L1) is greater than or equal to 20° and smaller than or equal to 45°.

11. The processing method according to claim 9, wherein
energy of the first laser (L1) is greater than that of the second laser (L2), and an area of the first laser (L1) is greater than an area of the second laser (L2); and/or
an area of the third laser (L3) is greater than an area of the second laser (L2).

12. The processing method according to claim 8, wherein the conducting spraying on a connection surface (12) of a ceramic substrate (10), so as to form a metal coating (20) on the connection surface (12) comprises:
conducting spraying on a center of the connection surface (12) so as to obtain a central zone (22); and
conducting spraying outwards from the central zone (22) so as to sequentially obtain a plurality of annular zones (23) located outside the central zone (22), wherein a thickness variation of the ceramic substrate (10) corresponding to a same annular zone (23) is smaller than or equal to 0.2 mm, wherein
the central zone (22) and the plurality of annular zones (23) constitute the metal coating (20).

13. The processing method according to claim 12, wherein the conducting spraying outwards from the central zone (22) so as to sequentially obtain a plurality of annular zones (23) located outside the central zone (22) comprises:
shortening spraying time and/or interval time of the plurality of annular zones (23) sequentially.
